# EUROPEAN PATENT SPECIFICATION

(11) **EP 4 161 614 B1**
(45) Date of publication and mention of the grant of the patent: **06.08.2025**
(21) Application number: 21818851.4
(22) Date of filing: 04.06.2021
(51) Int. Cl.: A61M 16/00

(54) **FEEDBACK PROVIDING FACIAL MASKS**
FEEDBACK ZUR BEREITSTELLUNG VON GESICHTSMASKEN
MASQUES FACIAUX FOURNISSANT UNE RÉTROACTION

(30) Priority: 04.06.2020 US 202063034561 P
(43) Date of publication of application: 12.04.2023
(73) Proprietor: University of Cincinnati, Cincinnati, OH 45206 (US)
(72) Inventor: MCMULLAN, Jason, Cincinnati, Ohio 45217 (US); BENOIT, Justin, Cincinnati, Ohio 45208 (US); GUTMARK, Ephraim J., Cincinnati, Ohio 45236 (US)
(74) Representative: Keltie LLP
(86) International application number: PCT/US2021/035918
(87) International publication number: WO 2021/248009

(56) References cited:
- EP-A1- 2 478 839
- WO-A1-2015/002652
- US-A1- 2016 106 941
- US-A1- 2017 065 784
- US-A1- 2017 361 045
- US-A1- 2018 310 893
- US-A1- 2019 275 357
- US-B1- 7 040 319
- US-B2- 10 065 055

## Description

### CROSS REFERENCE TO RELATED APPLICATION

This application claims benefit of U.S. Provisional Application No. 63/034,561 filed on June 4, 2020.

### TECHNICAL FIELD

The embodiments described herein generally relate to facial mask based oxygen delivery systems, and more specifically, to facial masks that are configured to form seals between the masks and faces of individuals and illuminate portions of these facial masks upon the formation of these seals.

### BACKGROUND

One of the biggest challenges for first responders, paramedics, and other medical personnel is providing patients with certain essential medical services in an effective and comprehensive manner. For example, first responders may arrive at the location of a critically ill patient in a timely manner and begin administrating various critical medical services, e.g., checking pulse rate, heart rate, providing oxygen, etc. However, conventional devices used to provide oxygen have numerous limitations. In particular, these include an inability to form an air-tight seal between the face mask and the face of an individual, which causes oxygen to escape from gaps existing between the location on an individual's face on which the facial mask is positioned and the oxygen delivery port or area of the facial mask via which oxygen is delivered to an individual. US2017/065784 A1, EP2478 839 A1, WO2015/002652 A1, US2017/361045 A1, US2016/106941 A1 disclose therapeutic fluid delivery devices, including, for example, therapeutic masks with sealing force detection.

Accordingly, a need exists for facial mask based oxygen delivery systems that enable first responders to provide adequate oxygen to patients via forming airtight seals between the masks and the faces of these patients, while informing these responders in real time of the strength of the seals between the masks and these patients.

### SUMMARY

The invention is defined by the appended claims. In one example, a facial mask that serves as a seal and an oxygen delivery device is provided. The mask includes a facial adaptive component including a deformable member embedded in an interior region of the facial adaptive component and a plurality of circuits, each of the plurality of circuits being operable in an open-circuit state or a closed-circuit state, a battery, a plurality of light elements coupled to the battery and positioned within the deformable member, a plurality of sensors positioned at a plurality of locations within the deformable member, each of the plurality of sensors connected to one of the plurality of light elements, wherein each of the plurality of circuits include the battery, one of the plurality of light elements, and at least one of the plurality of sensors, and wherein one or more of the plurality of circuits change from operating in the open-circuit state to the closed-circuit state in response to the one or more of the plurality of sensors contacting skin on an object that is external to the mask.

In another example, the mask includes a facial adaptive component including a deformable member embedded in an interior region of the facial adaptive component, a plurality of light elements coupled to a plurality of piezoelectric elements and positioned within the deformable member, and the plurality of piezoelectric elements positioned at a plurality of locations within the deformable member, wherein each of the plurality of piezoelectric elements provides electrical power to a respective one of the plurality of light elements in response to each of the plurality of piezoelectric elements being pressed against skin on an object that is external to the mask.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1 depicts an example design of a feedback providing facial mask of the present disclosure, according to one or more embodiments described and illustrated herein;
FIG. 2 depicts an example design of a feedback providing facial mask with a plurality of circuits embedded at various locations within the deformable member of the facial adaptive component of the mask, according to one or more embodiments described and illustrated herein;
FIG. 3A depicts an example configuration of an operation of a circuit of the plurality of included circuits included as part of the feedback providing facial mask of the present disclosure, according to one or more embodiments described and illustrated herein;
FIG. 3B depicts another example configuration of an operation of a plurality of circuits included as part of the feedback providing facial mask of the present disclosure, according to one or more embodiments described and illustrated herein;
FIG. 4A depicts an example operation of the feedback providing facial mask on the face of an example patient such that the plurality of circuits of the mask are operating in an open-circuit state, according to one or more embodiments described and illustrated herein; and
FIG. 4B illustrates an example operation of the feedback providing facial mask on the face of an example patient such that the plurality of circuits of the mask are operating in a closed-circuit state, according to one or more embodiments described and illustrated herein.

### DETAILED DESCRIPTION

The embodiments disclosed herein describe a feedback providing facial mask that enables an efficient and accurate determination of the presence of a seal between portions of the mask and the areas on the face of an individual upon which the mask is positioned. Conventionally, facial masks that are utilized to deliver oxygen to patients has numerous deficiencies. Specifically, when first responders place conventional facial masks on the faces of individuals, these responders have to rely on subjective and inaccurate techniques to determine whether the mask is providing sufficient oxygen to these individuals.

Some of these subjective methods include observing the sound of air reverberating across the cheeks of the individuals, etc. Additionally, obstacles to achieving a good seal include patient characteristics such as, e.g., presence of facial hair, lack of experience on the part of the first responder (e.g. holding the mask with one hand as opposed to two hands, etc.), etc. The lack of a proper seal between the mask and the face of an individual may result in insufficient delivery of oxygen to an individual, which may be result in poor tissue oxygenation, acid-base imbalance, and death. The feedback providing facial mask as described in the present disclosure addresses and overcome these deficiencies. Specifically, the feedback providing facial mask of the present disclosure enables first responders to efficiently and accurately determine the presence or absence of a seal (e.g., an air-tight seal) between the mask and the face of the individual upon whom the mask is positioned, namely via illuminating one or more of a plurality of light components and/or using audio components. In this way, the feedback providing facial mask can increase the amount of oxygen delivered to patients, thereby ensuring a better provision of medical services to these individuals.

As illustrated, the example feedback providing facial mask 101 includes an external adaptive component 102, a rigid component 104, and a facial adaptive component 106. In embodiments, the rigid component 104 may be disposed or positioned between the external adaptive component 102 and the facial adaptive component 106. In embodiments, the external adaptive component may be port or opening designed to have a circular, hexagonal, or other shape. It is noted that the external adaptive component 102 may be universal in that the opening may be designed to couple with an oxygen source having different specifications. For example, the external adaptive component 102 may couple with a portion of a self-inflating bag, a tube attached to an external oxygen source (e.g., an oxygen container), and so forth. In embodiments, such a tube may be rigid, flexible, and so forth. In embodiments, the external adaptive component 102 may be flexible and designed of rubber, plastic, etc.

In examples, the rigid component 104 may be designed to have a hollow portion. Additionally, the external adaptive component 102 may be detachably or permanently configured on one end of the rigid component 104 and the facial adaptive component 106 may be positioned on another end of the rigid component 104. The rigid component 104 may be formed of a hard plastic material such as high-density polyethylene, low-density polyethylene, or other comparable material. The hollow portion may be designed such that the rigid portion may be positioned upon and conform to the bridge of the nose and chin of an individual. In embodiments, the rigid component 104 may include a narrow end 108 (e.g., narrow portion) that may be aligned with, conform to, and positioned on the nose of an individual and wide end 110 (e.g., wide portion) that may be aligned with, conform to, and positioned on the chin of the individual. In embodiments, the facial adaptive component 106 may include a deformable member that is embedded within the facial adaptive component 106. For example, the deformable member may include or be formed of silicone material. Additionally, the facial adaptive component 106 may be formed of a flexible plastic material or cushion that facilitates comfortable positioned on the face of an individual.

In examples, the example feedback providing facial mask 101 may be designed such that, when attached to the face of an individual (e.g., a critically ill patient), portions of the facial adaptive component 106 directly contact areas on an individual's face extending from the bridge of the nose to the chin and approximately from a middle of one cheek to the middle of another cheek. The rigid component 104 may be positioned on top of the facial adaptive component, and the external adaptive component 102 may be detachably or permanently positioned on top of the rigid component 104. It is further noted that the external adaptive component 102, the rigid component 104, and the facial adaptive component 106 may be attached to each other via an adhesive, mechanical coupling components, and/or a combination thereof.

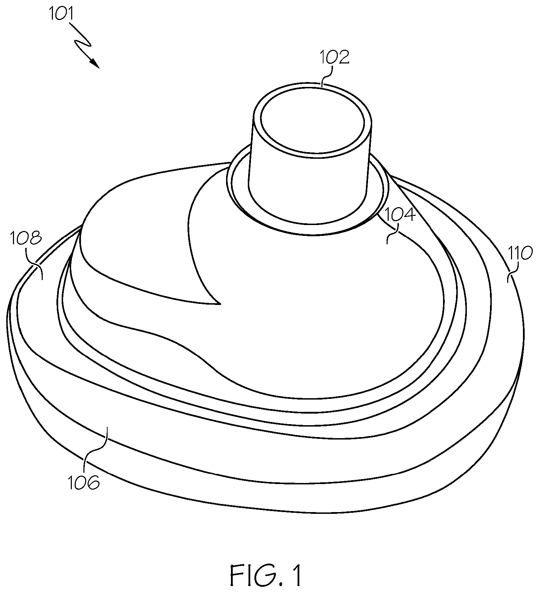

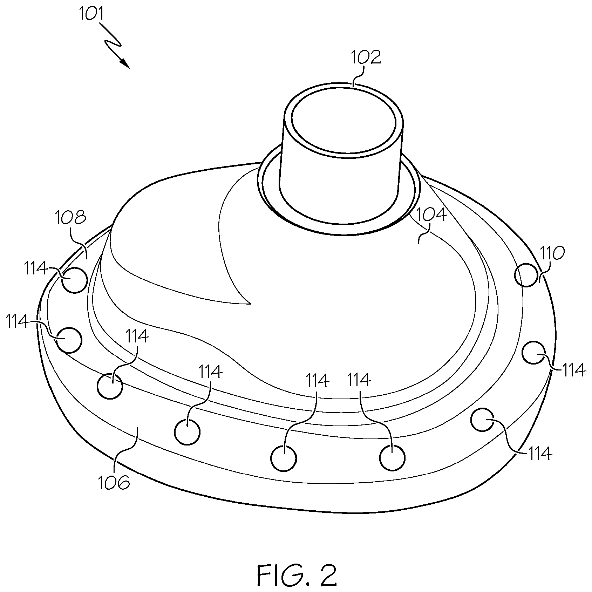

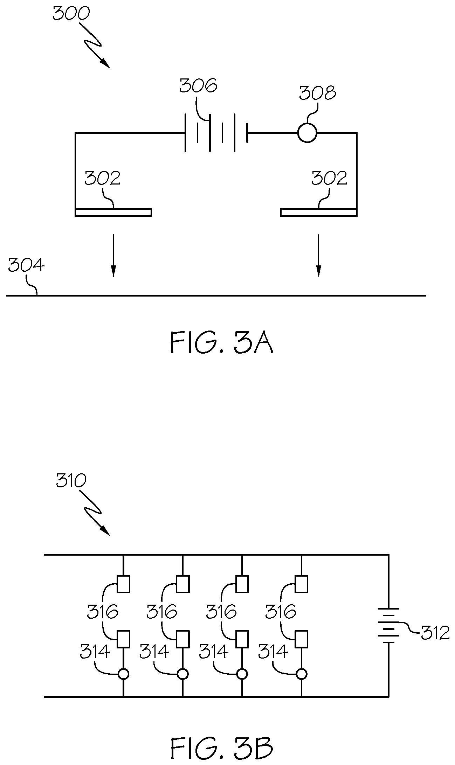

FIG. 2 depicts an example design of a feedback providing facial mask as described in FIG. 1 with a plurality of circuits embedded at various locations within the deformable member of the facial adaptive component 106, according to one or more embodiments described and illustrated herein. In embodiments, each of the plurality of circuits includes a battery, one or more light components 114, and one or more sensors. The battery is coupled to each of the light components 114 and each of the sensors and powers operation of the sensors and the light components 114. For example, the light components 114 may be connected to the battery in parallel as shown in FIG. 3B. It is noted that each of the plurality of circuits may operate in either a closed-circuit state or an open-circuit state. Each of the sensors may be embedded at various locations within the deformable member such that each of these sensors is configured to contact a separate and distinct portion on the face of the individual. Each of the lighting components may be light emitting diodes positioned in a particular portion of the deformable member, e.g., within a certain proximity of each sensor. Specifically, each light emitting diode may be positioned within a short distance of a corresponding sensor and be configured to operate in conjunction with the corresponding sensor.

In some examples, the feedback providing facial mask may include only a single circuit that may operate in a closed-circuit state or an open-circuit state. The single circuit may include a battery, a plurality of light components 114, and a plurality of sensors, all of which are connected in series.

During an example operation of the feedback providing facial mask, e.g., when the mask is positioned on a face of an individual, multiple sensors may directly come in contact with multiple areas on the face. Upon a sensor establishing a threshold level of contact with a portion of the skin, the circuit that includes this sensor may change operating from an open-circuit state to a closed-circuit state. Additionally, responsive to the change from the open-circuit state to the closed-circuit state, the light emitting diode adjacent to the sensor may be illuminated, e.g., by the battery. In other words, upon a threshold level of contact with the portion of skin on an individual's face, a clear path may form for current to flow from the cathode end of a battery to the anode end. As such, any components that are positioned along the path will be operable. Specifically, any and all light components located adjacent to these sensors will be illuminated by the current flow. It is noted that, in embodiments, the battery, the sensors, the skin portions with which the sensors contact, and the light components are all connected to each other in series.

In another example, an audio component (e.g., an alarm, buzzer, etc.) or a plurality of audio components may be embedded adjacent to each of the plurality of sensors and also be powered by the battery. Specifically, in this embodiment, the audio component, the sensors, the skin portions with which the sensors contact, and the lighting components may be connected in series as well. As such, in the closed-circuit state, the current flowing from the cathode end to the anode end may illuminate all lighting components located along the path of the current, in addition to powering the audio component such that the audio component outputs sound. It is also noted that, in this embodiment, the audio component may be operational based on tactile feedback. Additionally, in instances, a threshold level of pressure may be applied to a subset of the plurality of sensors, while a pressure less than the threshold level may be applied to another subset of the plurality of sensors.

In such a scenario, only a subset of the light components, namely the ones adjacent to or associated with the subset of the plurality of sensors on which the threshold level of pressure is applied may be illuminated. The other light components may not be illuminated. Specifically because the current path does not extend to all of the lighting components. Such a scenario or status may inform a first responder that a partial seal has been formed with respect to some of the circuits, but he or she needs to make adjustments to the position of the mask or increase pressure on some portions of the mask in order to form the seal with respect to the remaining circuits. In this way, the first responders may determine, in real time, whether seals are formed without having to rely on inaccurate and subject techniques such as observing or noticing the sound of air reverberating across the cheeks, and so forth.

In yet another example, the sensors may be a plurality of piezoelectric elements embedded within various locations in the deformable member along the boundary of the facial adaptive component 106. Additionally, each of the plurality of piezoelectric elements may include a lighting component (e.g., a light emitting diode) positioned within a certain proximity of the element. Each of these piezoelectric elements operates such that when a feedback providing mask is positioned on a face of an individual, one or more of the piezoelectric elements may contact various portions of the face of the individual. Upon contact with the face of an individual, each of the piezoelectric elements will experience a certain level of mechanical pressure.

The mechanical pressure will be utilized by the piezoelectric element to generate an output voltage that will power a particular light emitting diode positioned adjacent to or within a certain vicinity of the piezoelectric element. It is noted that the piezoelectric elements do not require an outside power source (e.g., a battery) to power them. Instead, these elements may utilize an internal charge amplifier (embedded within or installed as part of the piezoelectric element) that converts the mechanical strain or pressure applied on the piezoelectric element to a voltage amount that is output for and used to, e.g., illuminate a light emitting diode positioned nearby. As such, in this design or embodiment of the feedback providing facial mask, a battery may not be included.

FIG. 3A depicts an example configuration of an operation of a circuit of the plurality of circuits included as part of the feedback providing facial mask, according to one or more embodiments described and illustrated herein. Specifically, FIG. 3A depicts a schematic illustration of an example circuit 300 of the plurality of circuits during the point at which the circuit changes operating from an open-circuit state to a closed-circuit state. Upon the feedback providing facial mask being positioned on a face of an individual, the example sensors 302 of the circuit 300 may contact skin 304 located on the individual's face. Contact with the skin closes the state of the circuit 300 such that there is a clear path for the current to flow from the cathode end of the example battery 306 to the anode end of the example battery 306. In operation, the clear path for the current results in the current illuminating the example light emitting diode 308. The light emitting diode 308 may correspond to one of the light components 114 in FIG. 2. However, if there is gap or break in the current path, the current will be unable to flow from the cathode end of the example battery 306 to the anode end, thereby preventing the illumination of the example light emitting diode 308. In some embodiments, the light intensity of the light emitting diode 308 may be varied based on the degree of the contact between the sensors 302 and the skin 304. For example, when the sensors 302 are tightly pressed against the skin 304, the light emitting diode 308 may emit brighter light than the light emitted by the light emitting diode 308 when the sensors 302 are loosely pressed against the skin 304. As another example, when the entire surface of the sensors 302 contacts the skin 304, the light emitting diode 308 may emit brighter light than the light emitted by the light emitting diode 308 when 50% of the surface of the sensors 302 contact the skin 304.

In examples, such a gap may result for various reasons. For example, if a first responder or medical personnel fails to accurately place the feedback providing facial mask on an individual's face such that one or more of the sensors fail to contact skin, a gap may form between the sensor on the skin on the individual's face. Additionally, the gap will result in the lack of illumination of an LED located adjacent to and coupled to the sensor and a battery positioned near the sensor. As such, the first responder may, in real time, be able to determine the need to make an adjustment in the position of the feedback providing facial mask on the face of the individual.

In examples, in an example operation of the feedback providing facial mask, a subset of the circuits may be operating in a closed-circuit state and another subset of the circuits may be operating in an open-circuit state. The circuits operating in the closed-circuit state may not have a gap between the sensors of these circuits and the face of an individual upon which the mask may be positioned, and the circuits operating in an open-circuit state may have gaps between the sensors of these circuits and the face of an individual upon which the mask may be positioned.

Additionally, in examples, the subset of circuits that operate in a closed-circuit state are indicative of a presence of a seal (e.g., an air tight seal) between the portions of facial adaptive components in which the subset of circuits are located and a part of the face of an individual upon which the feedback providing mask is positioned. By contrast, the subset of circuits that operate in an open-circuit state are indicative of the lack of a presence of a seal (e.g., an air tight seal) between the portions of the facial adaptive component on which this subset of circuits are located and a part of the individual upon which the feedback providing mask is positioned. As such, first responders and medical personnel may view the illumination status of the plurality of light components (e.g., feedback provided by the mask) and determine, in real time, whether a seal exists between various parts of the mask and a patient's face, thereby ensuring that oxygen is delivered to a particular patient, namely without escaping through gaps between the mask and the patient's face.

FIG. 3B depicts another example configuration of an operation of a plurality of circuits included as part of the feedback providing facial mask of the present disclosure, according to one or more embodiments described and illustrated herein. Specifically, FIG. 3B depicts a plurality of example circuits 310 that are connected in parallel and configured to be powered by an example battery 312. Specifically, the plurality of example circuits 310 include the example battery 312, a plurality of example sensors 316, and a plurality of example light components 314 that are connected to the plurality of example sensors 316 in parallel. In embodiments, if each of the plurality of example sensors 316 contact various portions of an individual's face upon which there is skin, each of the plurality of example circuits 310 may change operation from an open-circuit state to a closed-circuit state. As described above, in the closed-circuit state, each of the plurality of example light components 314 may be illuminated because, based on the contact of the sensors with skin on the individual's face, a path may be available for the current to flow from the cathode end of the example battery 312 to the anode end of the example battery 312. As the current flows along this path, it will illuminate the plurality of example light components 314 and power operation of the plurality of example sensors 316. In other embodiments, audio components (e.g., a speaker, a buzzer, etc.) may be included within the plurality of example circuits 310. These audio components may also be powered by the example battery 312.

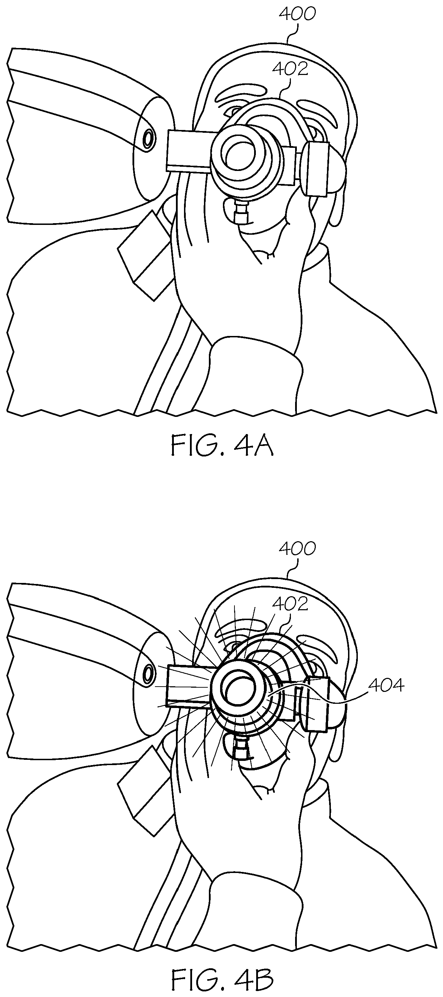

FIG. 4A depicts an example operation of the feedback providing facial mask on the face of an example patient 400 such that the plurality of circuits of the mask are operating in an open-circuit state, according to one or more embodiments described and illustrated herein. In an example operation, a first responder may receive a call from a critically ill patient (e.g., example patient 400) and travel to the location of the patient within a short time frame. Upon arrival, the first responder may determine that the example patient 400 is in immediate need of oxygen and may begin fitting the mask on the face of the example patient 400. However, if the example feedback providing facial mask 402 is lightly placed on top of the example patient 400, there may be gaps between the patient's face and the example facial adaptive component of the example feedback providing facial mask 402. Consequently, as illustrated in FIG. 4A, none of the light components of the example feedback providing facial mask 402 are illuminated. As previously described, one or more of the lighting components may fail to illuminate for a variety of other reasons, e.g., the first responder holding the example facial adaptive portion incorrectly over the face of the example patient 400 (e.g., only with one hand), not placing the middle portion of the mask directly over the mount of the example patient 400, and so forth. Other such reasons are contemplated.

FIG. 4B illustrates an example operation of the feedback providing facial mask on the face of an example patient 400 such that the plurality of circuits of the mask are operating in a closed-circuit state, according to one or more embodiments described and illustrated herein. A first responder, as described in FIG. 4A, may respond to an emergency call from a critically ill patient (e.g., the example patient 400), may arrive at the location of the patient, and begin administrating emergency medical services, including providing oxygen. In embodiments, as described in FIG. 4A, the first responder may position the feedback providing facial mask on the face of the critically ill patient in a particular manner and notice that none of the light components are illuminated. Such an operating state may, in real time, indicate to the first responder that there is a lack of a seal between the face of the example patient 400 and the example feedback providing facial mask 402 of the present disclosure.

In response, the first responder may adjust the position of the mask on the patient's face in order to ensure that a seal is formed. For example, the first responder may increase the pressure with which the example feedback providing facial mask 402 is placed on the face of patient's face, move the mask left, right, up, or down, etc., to make sure that the facial adaptive component of the example feedback providing facial mask 402 directly contacts areas on an individual's face extending from the bridge of the nose to the chin and approximately from a middle of one cheek to the middle of another cheek. Upon applying the requisite amount of pressure such that the sensors embedded in the facial adaptive component contacts various portions of the face of the example patient 400 having skin, the plurality of circuits in which these sensors are embedded close a path for current to flow from the cathode end of the battery to the anode end. The current illuminates any and all light components 404 (e.g., light emitting diodes) adjacent to these sensors, as illustrated in FIG. 4B. In this way, the first responder is notified, in real time, of the presence of, e.g., an air-tight seal between the example feedback providing facial mask 402 and the example patient 400.

Such a seal increases the amount of oxygen that is delivered to the example patient 400 via the mask by reducing any instance of oxygen leakage. Additionally, as described above, in another embodiments, an audio component (e.g., a buzzer) may be embedded within the facial adaptive component and be configured to output sound simultaneously with the illuminated light components. The audio component may be activated by tactile feedback and be powered by a battery. In embodiments, the audio component may be placed in series with the sensors and the light components such that the current that illuminates the light components may be utilized to operate the audio component.

It should be understood that the embodiments described herein relate to a feedback providing facial mask. The mask includes a facial adaptive component including a deformable member embedded in an interior region of the facial adaptive component and circuits, each of the circuits being operable in an open-circuit state or a closed-circuit state. The mask also includes a battery, light elements coupled to the battery and positioned within the deformable member, and sensors positioned at a plurality of locations within the deformable member. Each of the plurality of sensors connected to one of the light elements, wherein each of the circuits include the battery, one of the light elements, and at least one of the plurality of sensors, and wherein one or more of the circuits change from operating in the open-circuit state to the closed-circuit state in response to the one or more of the sensors contacting skin on an object that is external to the mask.

The terminology used herein is for the purpose of describing particular aspects only and is not intended to be limiting. As used herein, the singular forms "a," "an," and "the" are intended to include the plural forms, including "at least one," unless the content clearly indicates otherwise. "Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof. The term "or a combination thereof" means a combination including at least one of the foregoing elements.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A mask comprising:
a facial adaptive component (106) including a deformable member embedded in an interior region of the facial adaptive component (106) and a plurality of circuits (310), each of the plurality of circuits being operable in an open-circuit state or a closed-circuit state;
a battery (312);
a plurality of light elements (114) coupled to the battery and positioned within the deformable member; and
a plurality of sensors (316) positioned at a plurality of locations within the deformable member, each of the plurality of sensors connected to one of the plurality of light elements,
wherein each of the plurality of circuits include the battery, one of the plurality of light elements, and at least one of the plurality of sensors,
wherein one or more of the plurality of circuits change from operating in the open-circuit state to the closed-circuit state in response to one or more of the plurality of sensors contacting skin on an object that is external to the mask; and
**characterized in that** the plurality of light elements emit varied light intensity based on a degree of contact between the plurality of sensors and the skin.

2. The mask of claim 1, wherein the closed-circuit state of the plurality of circuits is associated with a seal between the facial adaptive component and the object that is external to the mask.

3. The mask of claim 1, wherein at least a subset of the plurality of light elements are configured to illuminate, using the battery, responsive to at least one of the plurality of circuits changing from operating in the open-circuit state to the closed-circuit state.

4. The mask of claim 3, wherein the subset of the plurality of light elements are positioned adjacent to and coupled with the subset of the plurality of sensors contacting skin on the object that is external to the mask.

5. The mask of claim 1, wherein each of the plurality of light elements is coupled to a respective sensor of the plurality of sensors.

6. The mask of claim 1, wherein each of the plurality of light elements is a light emitting diode that is powered by the battery.

7. The mask of claim 1, wherein the object that is external to the mask is a face of an individual.

8. The mask of claim 7, wherein the facial adaptive component includes a narrow portion that conforms to a nose on the face and a wide portion that conforms to a chin on the face.

9. The mask of claim 1, further comprising a rigid component disposed between the facial adaptive component and an external adaptive component.

10. The mask of claim 1, further comprising an audio component.

11. The mask of claim 10, wherein the audio component emits sound responsive to one or more of the plurality of circuits changing from operating in the open-circuit state to the closed-circuit state.

12. The mask of claim 1, wherein the deformable member includes a silicone material.

## Patentansprüche

1. Eine Maske, die Folgendes umfasst:
eine gesichtsadaptive Komponente (106), die ein in einer inneren Region der gesichtsadaptiven Komponente (106) eingebettetes verformbares Glied und eine Vielzahl von Schaltungen (310) beinhaltet, wobei jede der Vielzahl von Schaltungen in einem Geöffnete-Schaltungs-Zustand oder einem Geschlossene-Schaltungs-Zustand betriebsfähig ist;
eine Batterie (312);
eine Vielzahl von Lichtelementen (114), die mit der Batterie gekoppelt und in dem verformbaren Glied positioniert sind; und
eine Vielzahl von Sensoren (316), die an einer Vielzahl von Stellen in dem verformbaren Glied positioniert sind, wobei jeder der Vielzahl von Sensoren mit einem der Vielzahl von Lichtelementen gekoppelt ist,
wobei jede der Vielzahl von Schaltungen die Batterie, eines der Vielzahl von Lichtelementen und mindestens einen der Vielzahl von Sensoren beinhaltet,
wobei eine oder mehrere der Vielzahl von Schaltungen als Reaktion darauf, dass einer oder mehrere der Vielzahl von Sensoren Haut auf einem Objekt kontaktieren, das extern von der Maske liegt, von dem Geöffnete-Schaltungs-Zustand in den Geschlossene-Schaltungs-Zustand wechselt; und
**dadurch gekennzeichnet, dass** die Vielzahl von Lichtelementen eine veränderliche Lichtintensität basierend auf einem Grad des Kontakts zwischen der Vielzahl von Sensoren und der Haut emittieren.

2. Maske nach Anspruch 1, wobei der Geschlossene-Schaltungs-Zustand der Vielzahl von Schaltungen mit einer Dichtung zwischen der gesichtsadaptiven Komponente und dem Objekt, das extern von der Maske liegt, assoziiert ist.

3. Maske nach Anspruch 1, wobei mindestens ein Teilsatz der Vielzahl von Lichtelementen dazu konfiguriert ist, als Reaktion darauf, dass mindestens eine der Vielzahl von Schaltungen von dem Betrieb in dem Geöffnete-Schaltungs-Zustand in den Geschlossene-Schaltungs-Zustand wechselt, unter Verwendung der Batterie aufzuleuchten.

4. Maske nach Anspruch 3, wobei der Teilsatz der Vielzahl von Lichtelementen neben dem Teilsatz der Vielzahl von Sensoren, die Haut auf einem Objekt kontaktieren, das extern von der Maske liegt, positioniert und damit gekoppelt ist.

5. Maske nach Anspruch 1, wobei jedes der Vielzahl von Lichtelementen mit einem jeweiligen Sensor der Vielzahl von Sensoren gekoppelt ist.

6. Maske nach Anspruch 1, wobei jedes der Vielzahl von Lichtelementen eine lichtemittierende Diode ist, die von der Batterie gespeist wird.

7. Maske nach Anspruch 1, wobei das Objekt, das außerhalb der Maske liegt, ein Gesicht eines Individuums ist.

8. Maske nach Anspruch 7, wobei die gesichtsadaptive Komponente einen schmalen Abschnitt, der sich einer Nase auf dem Gesicht angleicht, und einen breiten Abschnitt, der sich einem Kinn auf dem Gesicht angleicht, beinhaltet.

9. Maske nach Anspruch 1, die ferner eine starre Komponente umfasst, die zwischen der gesichtsadaptiven Komponente und einer externen adaptiven Komponente angeordnet ist.

10. Maske nach Anspruch 1, die ferner eine Audiokomponente umfasst.

11. Maske nach Anspruch 10, wobei die Audiokomponente als Reaktion darauf, dass eine oder mehrere der Vielzahl von Schaltungen von dem Betrieb in dem Geöffnete-Schaltungs-Zustand in den Geschlossene-Schaltungs-Zustand wechselt, einen Ton emittiert.

12. Maske nach Anspruch 1, wobei das verformbare Glied ein Silikonmaterial beinhaltet.

## Revendications

1. Masque comprenant :
un composant adaptatif facial (106) comportant un organe déformable intégré dans une région intérieure du composant adaptatif facial (106) et une pluralité de circuits (310), chacun de la pluralité de circuits étant capable de fonctionner dans un état de circuit ouvert ou un état de circuit fermé ;
une batterie (312) ;
une pluralité d'éléments lumineux (114) couplés à la batterie et positionnés au sein de l'organe déformable ; et
une pluralité de capteurs (316) positionnés à une pluralité d'emplacements au sein de l'organe déformable, chacun de la pluralité de capteurs étant relié à l'un de la pluralité d'éléments lumineux,
dans lequel chacun de la pluralité de circuits comporte la batterie, l'un de la pluralité d'éléments lumineux, et au moins l'un de la pluralité de capteurs,
dans lequel un ou plusieurs de la pluralité de circuits passe d'un fonctionnement dans l'état de circuit ouvert à l'état de circuit fermé en réponse à l'entrée en contact d'un ou de plusieurs de la pluralité de capteurs avec de la peau sur un objet qui est externe au masque ; et
**caractérisé en ce que** la pluralité d'éléments lumineux émettent une intensité de lumière variable sur la base d'un degré de contact entre la pluralité de capteurs et la peau.

2. Masque selon la revendication 1, dans lequel l'état de circuit fermé de la pluralité de circuits est associé à un joint formé entre le composant adaptatif facial et l'objet qui est externe au masque.

3. Masque selon la revendication 1, dans lequel au moins un sous-ensemble de la pluralité d'éléments lumineux sont configurés pour illuminer, à l'aide de la batterie, en réponse au passage par au moins l'un de la pluralité de circuits d'un fonctionnement dans l'état de circuit ouvert à l'état de circuit fermé.

4. Masque selon la revendication 3, dans lequel le sous-ensemble de la pluralité d'éléments lumineux sont positionnés adjacents et couplés au sous-ensemble de la pluralité de capteurs en contact avec de la peau sur l'objet qui est externe au masque.

5. Masque selon la revendication 1, dans lequel chacun de la pluralité d'éléments lumineux est couplé à un capteur respectif de la pluralité de capteurs.

6. Masque selon la revendication 1, dans lequel chacun de la pluralité d'éléments lumineux est une diode électroluminescente qui est alimentée par la batterie.

7. Masque selon la revendication 1, dans lequel l'objet qui est externe au masque est un visage d'un individu.

8. Masque selon la revendication 7, dans lequel le composant adaptatif facial comporte une portion étroite qui épouse un nez sur le visage et une portion large qui épouse un menton sur le visage.

9. Masque selon la revendication 1, comprenant en outre un composant rigide disposé entre le composant adaptatif facial et un composant adaptatif externe.

10. Masque selon la revendication 1, comprenant en outre un composant audio.

11. Masque selon la revendication 10, dans lequel le composant audio émet un son en réponse au passage par un ou plusieurs de la pluralité de circuits d'un fonctionnement dans l'état de circuit ouvert à l'état de circuit fermé.

12. Masque selon la revendication 1, dans lequel l'organe déformable comporte un matériau de silicone.
